# EUROPEAN PATENT SPECIFICATION

(11) **EP 4 159 842 B1**
(45) Date of publication and mention of the grant of the patent: **05.08.2026**
(21) Application number: 20937954.4
(22) Date of filing: 01.06.2020
(51) Int. Cl.: A01N 1/125, C12N 5/0775, A61K 35/28, C12N 5/00

(54) **METHOD FOR IN VITRO SCREENING, ACTIVATION, PROLIFERATION, AND CRYOPRESERVATION OF MESENCHYMAL STROMAL/STEM CELLS AND MESENCHYMAL STROMAL/STEM CELL BANK ESTABLISHMENT**
VERFAHREN FÜR IN-VITRO-SCREENING, AKTIVIERUNG, PROLIFERATION UND KRYOKONSERVIERUNG VON MESENCHYMALEN STROMA-/STAMMZELLEN UND MESENCHYMALE STROMA-/STAMMZELLENBANK
PROCÉDÉ IN VITRO DE CRIBLAGE, D'ACTIVATION, DE PROLIFÉRATION ET DE CRYOCONSERVATION DE CELLULES STROMALES/SOUCHES MÉSENCHYMATEUSES ET D'ÉTABLISSEMENT DE BANQUE DE CELLULES STROMALES/SOUCHES MÉSENCHYMATEUSES

(30) Priority: 25.05.2020 CN 202010448992
(43) Date of publication of application: 05.04.2023
(73) Proprietor: Qingdao Restore Biotechnology Co., Ltd., Qingdao, Shandong 266000 (CN)
(72) Inventor: ZHANG, Bingqiang, Qingdao, Shandong 266000 (CN); CHEN, Mengmeng, Qingdao, Shandong 266000 (CN)
(74) Representative: Cabinet Chaillot
(86) International application number: PCT/CN2020/093716
(87) International publication number: WO 2021/237762

(56) References cited:
- WO-A1-2019/164680
- CN-A- 102 660 501
- CN-A- 103 422 176
- CN-A- 103 422 176
- CN-A- 104 894 064
- CN-A- 106 754 683
- CN-A- 110 172 445
- US-A1- 2015 374 755
- N. LI: "-MSC differentiation into male germ-like cells in vitro", CELL PROLIFERATION, vol. 47, no. 4, 13 June 2014 (2014-06-13), GB, pages 299 - 309, XP093159415, ISSN: 0960-7722, DOI: 10.1111/cpr.12115
- THORPE S D ET AL: "Dynamic compression can inhibit chondrogenesis of mesenchymal stem cells", BIOCHEMICAL AND BIOPHYSICAL RESEARCH COMMUNICATIONS, ELSEVIER, AMSTERDAM NL, vol. 377, no. 2, 12 December 2008 (2008-12-12), pages 458 - 462, XP025646096, ISSN: 0006-291X, [retrieved on 20081011], DOI: 10.1016/J.BBRC.2008.09.154
- TAN XIANG-LING ET AL: "5-(Hydroxymethyl)-2-furaldehyde inhibits adipogenic and enhances osteogenic differentiation of rat bone mesenchymal stem cells", NATURAL PRODUCT COMMUNICATIONS, NATURAL PRODUCT INC, US, vol. 9, no. 4, 1 April 2014 (2014-04-01), pages 529 - 532, XP009506948, ISSN: 1934-578X
- XI CHEN ET AL: "Bioreactor Expansion of Human Adult Bone Marrow-Derived Mesenchymal Stem Cells", STEM CELLS, vol. 24, no. 9, 1 September 2006 (2006-09-01), pages 2052 - 2059, XP055050653, ISSN: 1066-5099, DOI: 10.1634/stemcells.2005-0591
- ERTAO CHAO: "Effects of paeoniflorin on the proliferation of bone marrow mesenchymal stem cells", CHINESE JOURNAL OF TISSUE ENGINEERING RESEARCH, 1 January 2015 (2015-01-01), pages 101 - 107, XP093159593, Retrieved from the Internet <URL:https://pesquisa.bvsalud.org/portal/resource/pt/wpr-460892>
- MIN-JI AHN ET AL: "Metformin promotes neuronal differentiation and neurite outgrowth through AMPK activation in human bone marrow-mesenchymal stem cells", BIOTECHNOLOGY AND APPLIED BIOCHEMISTRY, ACADEMIC PRESS, US, vol. 64, no. 6, 7 September 2017 (2017-09-07), pages 836 - 842, XP071713550, ISSN: 0885-4513, DOI: 10.1002/BAB.1584
- GEORGE SAJAN ET AL: "Differentiation of Mesenchymal Stem Cells to Neuroglia: in the Context of Cell Signalling", STEM CELL REVIEWS AND REPORTS, SPRINGER US, NEW YORK, vol. 15, no. 6, 12 September 2019 (2019-09-12), pages 814 - 826, XP036975818, ISSN: 2629-3269, [retrieved on 20190912], DOI: 10.1007/S12015-019-09917-Z
- CHAO ER-TAO; BAI HAI; WANG CUN-BANG; XI RUI; OU JIAN-FENG: "Effects of Paeoniflorin on the Proliferation of Bone Marrow Mesenchymal Stem Cells", CHINESE JOURNAL OF TISSUE ENGINEERING RESEARCH, vol. 19, no. 1, 1 January 2015 (2015-01-01), pages 101 - 107, XP009532117

## Description

### Technical Field

The present invention relates to a method for screening, activating, amplifying and cryopreserving mesenchymal stem cells in vitro and establishing a cell bank of mesenchymal stem cells.

### Background

As the greatest hotspot of biological world in recent years, the development of stem cells will provide revolutionary technical means in medical field. Stem cell is a kind of primitive cell having self replication capacity and multi-lineage differentiation. In certain conditions, the stem cell can be differentiated into a variety of functioning cells, and can be used for treating multiple diseases, such as, leukemia, congenital metabolic diseases, certain solid tumors, diabetes, heart disease and cerebral palsy and thus, has very broad medical purposes. There are more than 220 kinds of cells in a human body; these cells are organically integrated into complex tissues and organs; each cell has its specific functions, such as, contraction of myocardial cells, information transfer functions of nerve cells, and the like. Stem cell is the progenitor cell of these cells, and also called "universal cell" in medical field.

Mesenchymal stromal/stem cells (MSCs) are derived from mesoderm and ectoderm in early development, and belong to multipotential stem cells and have multi-lineage differentiation. Further, MSC can be differentiated into multiple histocytes, such as, adipose, bone, cartilage, muscle, tendon, ligamentum, nerve, liver, cardiac muscle, and endothelium. In 2006, MSC was defined by International Society for Cellular Therapy (ISCT). The cells conforming to the three standards simultaneously can be called MSC: ① adherence growth; ② some specific antigens (markers) are expressed on the cell surface; ③ ability to differentiation into adipocyte, osteoblast and chondrocyte. MSC is the adult stem cell which is most safe and effective and has wide application in therapy aspect at present, and is mainly derived from bone marrow, adipose, umbilical cord, placenta, amnion and the like. Compared with stem cells, MSC has the advantages, such as, easy accessibility, easy culture in vitro, long-term and stable subculture, low immunogenicity, and strong tissue repair capacity. Moreover, MSC is from adult cells, and can be even obtained from a patient's body instead of an embryo or fetal stem cells and thus, is not involved to morality and ethics problems. After through continuous subculture and cryopreservation, MSC still has the potential of multilineage differentiation and self replication. Researches show that MSC of human bone marrow can still keep the properties of stem cells after being subcultured for 40 years above in vitro.

MSC has more functions and wide applications, and has a major function of cell transplantation therapy, and can be also used as an ideal target cell for gene therapy. Meanwhile, MSC has certain applications in tissue engineering and immunotherapy. In recent years, a large number of MSC have been applied in experiments and clinical studies; a great number of studies have revealed the application value of MSC in the diagnosis and treatment of systemic diseases such as, cardiovascular system, nervous system, motor system, digestive system, autoimmune disease, blood system, urinary system, ophthalmology department, orthopedics department. In 2016, China published the first batch of 30 clinical research organizations for stem cells. Since 2019, dynamic management has been implemented in clinical research organizations for stem cells and project filing; up to September 2019, the number of clinical research hospitals for stem cells approved by China have increased to 106, and the number of approved hospitals of the military system has increased to 12; there are 118 organizations in total; further, the filed projects have increased to 62; document researches and patent applications grow continuously. Up to March 2020, 5432 clinical researches related to stem cells have been registered on ClinicalTrial.gov, including 469 of China. More researches are carried out in Guangzhou, Beijing and Shanghai.

Patent application CN103422176A discloses a method for constructing human amniotic mesenchymal stem cell bank. The screening medium of the present application is not disclosed.

However, the key to MSC therapy is to obtain higher-quality cells. The challenge to stem cell therapy lies in complex stem cell products and great influences of the difference of cell sources and production technologies on quality and therapeutic effects of stem cells, which is the major cause of non-ideal clinical trial results treated by numerous stem cells. Therefore, how to obtain a large number of standardized, high-quality and highly active MSC becomes the most important factor to restrict the development of cell industry. The method for in vitro screening, activation, amplification, cryopreservation and bank establishment of MSC needs to be improved.

### Summary

The present invention is aimed at solving the technical problem existing in the prior art. For the purpose, the present invention puts forward a method for screening, activating, amplifying and cryopreserving mesenchymal stem cells in vitro and establishing cell bank of mesenchymal stem cells. The method of screening, activating and amplifying MSCs has high efficiency, fast speed, high safety and low cost. Moreover, a cell bank can be established for a large number of functionally activated MSCs obtained by screening and amplification for long-stem storage; and after resuscitation, the MSC can still keep good cell viability.

The present invention provides a method for screening, activating, amplifying and cryopreserving mesenchymal stem cells in vitro and establishing a cell bank of mesenchymal stem cells. Based on the examples of the present invention, the method includes the following steps:
using a dedicated primary screening medium of mesenchymal stem cells for first-stage screening culture to obtain purified MSC; using a dedicated activation and amplification medium of mesenchymal stem cells to perform second-stage activation and large-scale amplification culture on the purified MSC to obtain a large number of mesenchymal MSC with activation function; using a dedicated cryopreserving fluid of mesenchymal stem cells to cryopreserve the stem cells and performing preservation according to ABO/RH typing and HLA typing; and establishing an information file for retrieval to construct a mesenchymal stem cell bank.

The dedicated primary screening medium of mesenchymal stem cells is a serum-free complete medium added with 2-8 ng/ml SCF, 2-4 ng/ml BMP-4, 10-30 IU/ml IL-10 and 1-4 ng/ml LIF, 1-4 ng/ml TGF-β, 2-8 ng/ml rapamycin, 2-12 ng/mlTrametinib , 10-20 ng/ml paracetamol, 1-3 ng/ml 5-HMF and 10-20 ng/ml chloroquine phosphate.

The dedicated activation and amplification medium of mesenchymal stem cells is a serum-free complete medium of mesenchymal stem cells added with 2-8 ng/ml SCF, 1-4 ng/ml bFGF, 10-20 ng/ml paeoniflorin, 20-30 ng/ml metformin hydrochloride, 1-4 ng/ml Hydrocortisone , 2-4 ng/ml CXCL10, 1-2 ng/ml Forskolin, 1-3 ng/ml 5-HMF, and 10-20 ng/ml chloroquine phosphate.

The dedicated cryopreserving fluid of mesenchymal stem cells is a 66-83 vol% serum-free complete medium of mesenchymal stem cells containing 5-10 vol% DMSO, 5-10 vol% multiple electrolytes injection, 5-10 vol% hydroxyethyl starch 200/0.5 sodium chloride injection, 1-2 vol% albumin, and 1-2 vol% hydroxylcamptothecine injection.

In the dedicated primary screening medium of mesenchymal stem cells, the dedicated activation and amplification medium of mesenchymal stem cells and the dedicated cryopreserving fluid of mesenchymal stem cells, the serum-free complete medium is a TheraPEAK^{™} MSCGM-CD^{™} Medium, MesenPRO RS^{™} Medium, Corning^{®} MSC Xeno-Free SFM or other types of commercially available serum-free media.

In the method of the present invention, the mesenchymal stem cell culture is divided into the first-stage screening culture and the second-stage activation and large-scale amplification culture; different culture conditions are used in the two stages.

The first-stage screening culture is mainly to remove parenchyma cells in the primary passage. MSCs derived from bone marrow, umbilical cord and placenta will be blended with more or less blood cells, endothelial cells and other parenchyma cells in the primary preparation process. The dedicated primary screening medium of mesenchymal stem cells is particularly added with cell promoting growth factors and screening factors. Recombinant Human Stem Cell Factor (SCF) can stimulate cell proliferation and migration in vitro. Recombinant Human Bone Morphogenetic Protein 4 (BMP-4) is a kind of effective bone morphogenetic protein, and is a portion of transforming growth factor (TGF-β) superfamily and works in the formation of mesenchymal cells and the development process of multiple organs. Paracetamol is a common antipyretic analgesic. It is found in the present invention that after a suitable concentration of paracetamol is added, non-MSCs in primary culture are inhibited obviously; the apoptosis rate of parenchyma cells is quickened, but MSC is free of influence. Rapamycin is a specific mTOR inhibitor with IC50 of 0.1 nM; rapamycin exerts anti-tumor effect via induction and autophagy to inhibit the vitality of tumor cells in a dose-dependent way. A low-dose rapamycin in this present invention can induce the non-MSC autophagy and apoptosis in primary passage. It is found in the present invention that a very low dose (not greater than 20 ng/ml) of chloroquine phosphate (conventional concentration is about 5 ug/ml, capable of being used as an autophagy inhibitor and a lysosome inhibitor) can significantly promote the proliferation of MSCs and can serve as an autophagy inhibitor against the rapamycin-induced autophagy. Trametinib is an extracellular signal-regulated kinase (MEK1) activated by mitogen and a reversible inhibitor activated by MEK2 and having MEK1 and MEK2 kinase activity. MEK protein is an upstream regulator on an the extracellular signal molecule-related kinase (ERK) pathway to promote cell proliferation. 5-Hydroxymethylfurfural (5-HMF) has antioxidation to resist oxidative damage caused by hydrogen peroxide; and its mechanism of action may be related that 5-HMF reduces nuclear factor κB protein expression and increases Bcl-2 protein expression.

The second stage is activation and large-scale amplification culture. SCF and basic fibroblast growth factor (bFGF) can stimulate cell proliferation and migration in vitro. Dimethyldiguanide has experienced a course of 50 years since its appearance. Dimethyldiguanide has pharmacological actions in various aspects of inhibiting liver glucose output and increasing the sensibility of peripheral organs on insulin and thus, has been widely applied in the treatment of type 2 diabetes, polycystic ovarian syndrome, obesity and other metabolic disorders. In recent years, researches have showed more and more functions of dimethyldiguanide. The present invention finds that metformin hydrochloride can activate MSCs, promote the proliferation and activization of stem cells, and significantly promote the secretion capacity of MSC factors. Paeoniflorin (PF) is the major active ingredient of a common traditional Chinese medicine, Chinese herbaceous peony, and is a kind of monoterpenes glucoside compound. In recent years, domestic and foreign scholars have carried out more thorough studies on the pharmacological action of paeoniflorin to find that paeoniflorin has the activity of anti-free radical injury, inhibiting intracellular calcium overload and anti-neurotoxicity, and to prove paeoniflorin has multiple biological effects of reducing blood viscosity, anti-platelet aggregation, dilation of blood vessels, improving microcirculation, antioxidation and anticonvulsion, and has low toxic and side effects via in vivo experiments. The present invention finds that paeoniflorin can activate MSCs, promote the proliferation and activization of stem cells, inhibit apoptosis of stem cells and significantly promote the secretion capacity of MSC factor. CXC chemokine ligand-10 (CXCL10), namely, interferon-inducible protein-10 (IP-10) can inhibit the formation of hematopoietic cell colony, perform monocyte chemotaxis, activate T cells and natural killer cells, stimulate T cells to adhere on endothelial cells and natural killer cell-mediated cytolysis, inhibit angiogenesis and the like. The present invention finds that CXCL10 can activate MSCs for the first and promote the amplification and activation of stem cells, inhibit the apoptosis of stem cells and significantly promote the secretion capacity of the MSC factors. Forskolin is a kind of common eukaryocyte adenylyl cyclase (AC) activator and is usually used for improving the level of cAMP in cytophysiology study. Forskolin can catalyze a subunit to directly activate adenylate cyclase (AC) to increase the level of cyclic adenosine monophosphate (cAMP). Document researches find that Forskolin can promote the proliferation of olfactory ensheathing cells cultured in vitro and also can induce stem cell differentiation. The present invention finds that a proper concentration of Forskolin can promote the proliferation and activization of MSCs, inhibit the apoptosis of stem cells and promote the expression of surface markers of MSCs.

The dedicated cryopreserving fluid of mesenchymal stem cells of the present invention is particularly added with 5-10 vol% multiple electrolytes injection, which can maintain the crystal osmotic pressure better. 5-10 vol% hydroxyethyl starch 200/0.5 sodium chloride injection and 1-2 vol% albumin are particularly added to maintain colloid osmotic pressure better. 1-2 vol% hydroxylcamptothecine injection is added to greatly promote the postresuscitation cell viability.

The present invention is used to perform screening, activation and amplification culture on MSCs to obtain a large number of standardized, high-quality and highly active MSCs. The present invention has the advantages of high screening efficiency, fast amplification speed, high safety and low cost. Moreover, the present invention establishes the corresponding cell bank, classifies the storage of large-scale stem cells and thus has long effective shelf time. After recovery, cells still keep good vitality; and the cell recovery rate is high. Thereby, the present invention satisfies the demand for a large amount of cells in clinic treatment.

### Brief Description of the Drawings

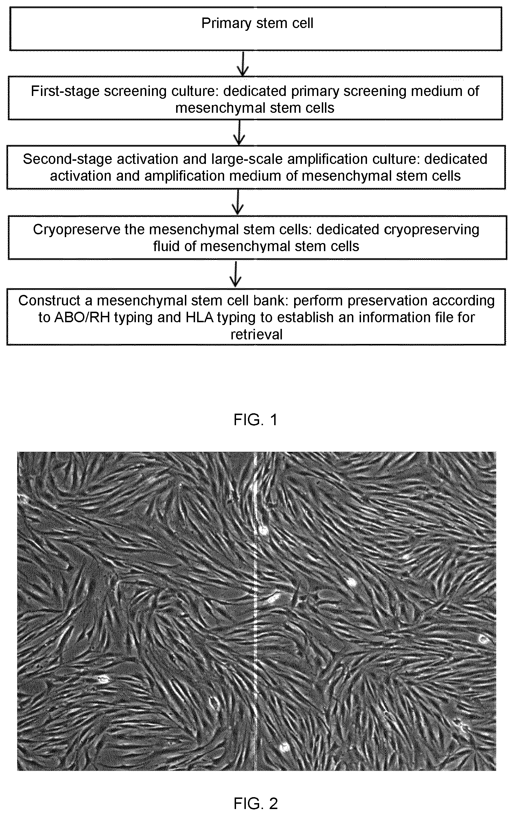

FIG. 1 is a flow diagram of the method of the present invention.
FIG. 2 is adipose MSCs (200×) obtained after culture for 14 d by the method the present invention.
FIG. 3 is a comparison of proliferation rate of stem cells between the method the present invention and a conventional culture method.

### Detailed Description of the Embodiments

The present invention provides a method for screening, activating, amplifying and cryopreserving mesenchymal stem cells in vitro and establishing cell bank of mesenchymal stem cells. The method includes the following steps: using a dedicated primary screening medium of mesenchymal stem cells for a first-stage screening culture to obtain purified MSCs; using a dedicated activation and amplification medium of mesenchymal stem cells to perform second-stage activation and large-scale amplification culture on the purified mesenchymal stem cells to obtain a large number of MSCs with activation functions; using a dedicated cryopreserving fluid of mesenchymal stem cells to cryopreserve the stem cells and performing preservation according to ABO/RH typing and HLA typing; and establishing an information file for retrieval to construct a mesenchymal stem cell bank.

According to the examples of the present invention, the dedicated primary screening medium of mesenchymal stem cells is a serum-free complete medium of mesenchymal stem cells added with 2-8 ng/ml SCF, 2-4 ng/ml BMP-4, 10-30 IU/ml IL-10 and 1-4 ng/ml LIF, 1-4 ng/ml TGF-β, 2-8 ng/ml rapamycin, 2-12 ng/mITrametinib, 10-20 ng/ml paracetamol, 1-3 ng/ml 5-HMF and 10-20 ng/ml chloroquine phosphate.

According to the examples of the present invention, the dedicated activation and amplification medium of mesenchymal stem cells is a serum-free complete medium of mesenchymal stem cells added with 2-8 ng/ml SCF, 1-4 ng/ml bFGF, 10-20 ng/ml paeoniflorin, 20-30 ng/ml metformin hydrochloride, 1-4 ng/ml Hydrocortisone, 2-4 ng/ml CXCL10, 1-2 ng/ml Forskolin, 1-3 ng/ml 5-HMF, and 10-20 ng/ml chloroquine phosphate.

According to the examples of the present invention, in the dedicated primary screening medium of mesenchymal stem cells, the dedicated activation and amplification medium of mesenchymal stem cells and the dedicated cryopreserving fluid of mesenchymal stem cells, the used serum-free complete medium is a TheraPEAK^{™} MSCGM-CD^{™} Medium, MesenPRO RS^{™} Medium, Corning^{®} MSC Xeno-Free SFM or other types of commercially available serum-free media.

According to the examples of the present invention, in the first-stage primary screening culture of mesenchymal stem cells, cells are subcultured once every 2-3 d for two times; in the second-stage activation and large-scale amplification culture of mesenchymal stem cells, cells are subcultured once every 2-3 d for more than once. Therefore, the MSC may achieve high-purity and large-scale amplification within a short time to obtain enough functionally activated MSCs, used for possible clinic treatment.

According to the examples of the present invention, the dedicated cryopreserving fluid of mesenchymal stem cells is a 66-83 vol% serum-free complete medium of mesenchymal stem cells containing 5-10 vol% DMSO, 5-10 vol% multiple electrolytes injection, 5-10 vol% hydroxyethyl starch 200/0.5 sodium chloride injection, 1-2 vol% albumin, and 1-2 vol% hydroxylcamptothecine injection. The mesenchymal stem cells have a cryopreservation concentration of (1×107-5×108)/ml. Therefore, the present invention has high cryopreserved cell concentration, low cryopreservation cost and good cell cryopreservation effect, high cell viability after resuscitation and cell yield, and is suitable for the cryopreservation of large-scale stem cells.

According to the examples of the present invention, the activated and amplified mesenchymal stem cells are preserved by ABO/RH typing and HLA typing to establish an information file of mesenchymal stem cells for retrieval and construct a mesenchymal stem cell bank.

The technical solution of the present invention will be explained in combination with examples below. Unless otherwise specified, the method used in the following examples is a conventional method; and the required reagents, consumable items and laboratory apparatus may be purchased via business approaches.

### Example 1

The method for screening, activating, and amplifying mesenchymal stem cells in vitro of the present invention was used to isolate and obtain adipose MSCs.
1. Donor screening: a collection hospital need to sign an informed consent with a donor, in triplicate. Two copies were kept by the donor and hospital, and the other one was submitted to the laboratory with a specimen. The hospital consulted the donor's personal information, history of treatment, history of familial inheritance, and whether of history of infectious diseases, abnormal conditions of hematopoiesis or immune system and other information by inquiry and a way of filling a form. Donor's physical examination information includes the following items: HIV antibody, hepatitis B surface antigen and antibody, hepatitis C antibody, cytomegalovirus antibody, Treponema pallidum antibody, transaminase, and the like. The informed consent, personal information acquisition table, examination information and the like need to be numbered for seal preservation. Any personnel in contact with data shall not reveal the privacy without the consent of the donor or its authorized officer. The cells should be kept by ABO/RH typing and HLA typing to establish a donor archival information base of stem cells for retrieval.
2. Preparation of adipose-derived stem cells: adipose for experimental use was taken from a donor receiving abdominal liposuction. Note: 5 ml peripheral blood was reserved for fast examination and blood grouping. 20 mL adipose-normal saline mixture was obtained and centrifuged in aseptic conditions, and washed twice with PBS to remove stupefacients and blood cells, thus obtaining adipose granules with higher purity. The adipose granules were digested with 0.1% collagenase on a constant temperature shaker for 60 min at 37°C and 1500 r/min, and centrifuged for 10 min to remove the indigested adipose tissues and grease in the upper layer; 200 um precipitate was resuspended and filtered with a filter, then centrifuged again; red blood cells were lysed by a red blood cell lysis buffer for 5 min and washed by a phosphate buffer solution twice to obtain SVF. Stromal Vascular Fraction (SVF) is, namely, a stromal vascular fraction, and is an effective ingredient extracted from adipose tissues autologously extracted from a patient and contains a variety of cells with repair functions; SVF is a cell population formed by endothelial cells, non-distinctive stroma cells, blood cells, histological macrophages, hemopoietic progenitor cells, and MSCs. The culture was performed by the three ways respectively, namely, a conventional serum-supplied medium (DMEM+10%FBS+8 ng/ml bFGF), and a serum-free medium MSCGM-CD^{™} according to the method of the present invention.

2.1 The culture of the conventional serum-supplied medium (DMEM+10%FBS+8 ng/ml bFGF): according to the SVF cell population; 20 ml (DMEM+10%FBS+8ng/ml bFGF) was added according to a density of 1.0×106/ml and inoculated to a T175 culture flask and placed in a carbon dioxide incubator with the culture conditions of (37±0.5)°C and a carbon dioxide volume fraction of (5±0.2)%. The medium change was performed for once every 2-3 d. When primary culture cells were up to 70%-80% fusion for 6 d around, the cells were subcultured for 4 times.

### 2.2. The culture of the serum-free medium TheraPEAK^{™} MSCGM-CD^{™} Medium (brand: LONZA and Art.No.: 00190632):

According to the SVF cell population; 20 ml (DMEM+10%FBS+8ng/ml bFGF) was added according to a density of 1.0× 106/ml and inoculated to a T175 culture flask and placed in a carbon dioxide incubator with the culture conditions of (37±0.5)°C and a carbon dioxide volume fraction of (5±0.2)%. The medium change was performed for once every 2-3 d. When primary culture cells were up to 80% fusion for 6 d around, the cells were subcultured for 4 times.

### 2.3. The culture method of the present invention:

2.3.1 According to the cell population of SVF, 20 ml of the dedicated primary screening medium of MSCs with a density of 1.0×106/ml was added, namely, a MSCGM-CD^{™} Medium added with 5ng/ml SCF, 5ng/ml BMP-4, 20IU/ml IL-10 and 2ng/ml LIF, 2ng/ml TGF-β, 5ng/ml rapamycin, 8ng/mlTrametinib, 10ng/ml paracetamol, 2ng/ml 5-HMF and 15ng/ml chloroquine phosphate, and inoculated to a T175 culture flask, and put to a carbon dioxide incubator; the culture conditions were as follows: 37±0.5°C, carbon dioxide volume fraction of 5±0.2%. The cells were observed every day and medium change was performed every 2-3 d according to the color of the medium. When primary culture cells were up to 70%-80% fusion for 5 d around, the cells were subcultured once.

2.3.2 Subculture was performed to change the culture system into a dedicated activation and amplification medium of mesenchymal stem cells, namely, a serum-free complete medium of mesenchymal stem cells added with 5ng/ml SCF, 2ng/ml bFGF, 10ng/ml paeoniflorin, 20ng/ml metformin hydrochloride, 2ng/mlHydrocortisone, 3ng/ml CXCL10, 1ng/ml Forskolin, 1ng/ml 5-HMF and 10ng/ml chloroquine phosphate, for continuous culture. The cells were observed every day and medium change was performed every 2-3 d according to the color of the medium.

2.4 When the cells were amplified for 14 d by the above culture methods, the obtained MSCs were totally recovered and subjected to cell counting to calculate a cell yield. 10 ml of the medium (cultured for 72 h) supernatant was preserved to perform the detection for secretory cell factors TGF-β, GM-CSF, IL-2,IL-10, VEGF, HGF and PDGF according to the Elisa method. The recovered cells were resuspended with 200 ml normal saline and added with 10 ml human serum albumin and mixed evenly, and 10 ml cell suspension was taken for detection, and the rest cells might be injected into a re-transfusion bag for back transfusion. 10 ml cell suspension was extracted from the re-transfusion bag for the following examination: mycoplasma, endotoxin, microorganism and virus (five items). The remaining cells were continuously cultured to 28 d.

2.5 Surface marker on MSCs was detected by a flow cytometry. The mesenchymal stem cells 1×105 (100ul) cultured by the three methods for 14 d were respectively added 20ul CD29, CD73, CD90, CD105, CD34, CD45, CD14 and HLA-DR antibodies, and mixed well, and incubated for 30 min in the dark and washed twice with PBS, and detected by Backman Clouter FC500 flow cytometry.

2.6 Comparison of cell proliferation rate: the collected adipose MSCs after being cultured for 14 d according to the above three methods were respectively taken, digested and made into a single-cell suspension, and the suspension was inoculated to a 96-well culture plate by 5× 104/cm2; per well was added with 100 µl of the above three culture solution respectively (the method of the present invention was the dedicated activation and amplification medium added with mesenchymal stem cells). Meanwhile, a list of culture plates were taken and added to a growth medium without adipose MSCs with 100 µl culture solution per well as a blank control group. 4 wells were taken every 12 h to measure an absorbance value (D570 value) at a 570 nm wavelength by a MTT colorimetry for consecutive three days, then a cell growth curve was drawn. The method was as follows: when the culture was terminated, 20 µl MTT solution (5 mg/ml concentrated solution) was added per well and incubated for 4 h at 37°C; supernatant was sucked and 150 µl DMSO was added per well and vibrated for 10 min at room temperature; 570 nm wavelength was selected and the mean value in the list of the blank control group was adjusted to 0; the D570 value of each well was measured on an ELISA detector to obtain a mean value. Time serves as a horizontal axis and D570 value serves as a longitudinal axis to draw a cell growth curve.

2.7 Nude mice carcinogenicity test: SPF-grade female BALB/c nude mice (4-6-week old, and weight: 18-20 g) were fed in a capped mouse cage in a laminar air flow rack; drinking water, standard feed and other substances in contact with the animals were sterilized. The above MSCs after being cultured for 28 d were subcutaneously inoculated on the ribs of the nude mice according to 3×107/0.2 ml, and labeled by picric acid to observe the tumor formation situation for 2 months.

### 3 Experimental result

3.1 The cell yield and cell viability of the mice after being amplified according to the three culture methods: 2×107 SVF were cultured on a conventional serum-supplied medium for 14 d to obtain 2.17×109 MSCs with a viability of 94.36%; 2.69×109 MSCs were obtained after being cultured on a serum-free medium MSCGM-CD^{™} for 14 d with a viability of 96.71%; and 4.35×109 MSCs were obtained after being cultured by the method of the present invention for 14 d with a viability of 98.73%. The yield of the MSCs cultured by the method of the present invention is much higher than that of the other existing two conventional culture methods.

3.2 The detection results of Elisa secretory factors in the medium supernatant after being cultured and amplified for 14 d by the three methods are shown in Table 1. The ability of cell factor secretion of the MSCs cultured by the method of the present invention is much higher than that of the other two conventional culture methods.

**Table 1 Detection results of the cell factors in the medium supernatant after being cultured for 14 d by the three methods**

| | TGF-β (pg/ml) | GM-CSF (ng/ml) | IL-2 (pg/ ml) | IL-10 (pg/ml) | VEGF (pg/ ml) | HGF (ng/ ml) | PDGF (ng/ ml) |
|---|---|---|---|---|---|---|---|
| Conventional serum-supplied medium | 436.40 | 46.29 | 30.43 | 36.10 | 21.61 | 0.60 | 0.04 |
| Serum-free culture | 194.18 | 53.95 | 35.02 | 35.47 | 20.94 | 0.52 | 0.02 |
| Cultured by the method of the present invention | 446.06 | 78.07 | 44.80 | 44.57 | 41.56 | 0.72 | 0.05 |

3.3 A testing platform was entrusted to detect the antibody hepatitis B surface antigen, hepatitis C antigen, HIV antibody, Treponema pallidum specific antibody, macrophage virus and mycoplasma, bacteria and endotoxin in the MSC suspension obtained after being cultured and amplified for 14 d by the three methods. The test results show negative. The above result indicates that the bath of MSCs is safe and there is no pollution in the culture process.

3.4 Flow cytometer result of the cells obtained after being cultured and amplified for 14 d by the three methods: the cells obtained after being cultured and amplified by the three methods conform to the characteristics of the MSC surface marker; positive indexes are CD29, CD73, CD90 and CD105 and the results are shown in Table 2; and the negative indexes are CD34, CD45, CD14 and HLA-DR and the results are shown in Table 3.

**Table 2 Positive index results of the flow cytometer after being cultured for 14 d by the three methods**

| | CD29 | CD73 | CD90 | CD105 |
|---|---|---|---|---|
| Conventional serum-supplied medium | 87.01% | 91.74% | 89.61% | 90.32% |
| Serum-free culture | 96.00% | 95.33% | 95.99% | 96.38% |
| Cultured by the method of the present invention | 99.10% | 99.36% | 99.51% | 99.03% |

**Table 3 Negative index results of the flow cytometer after being cultured for 14 d by the three methods**

| | CD34 | CD14 | HLA-DR | CD45 |
|---|---|---|---|---|
| Conventional serum-supplied medium | 1.85% | 1.94% | 1.52% | 1.71% |
| Serum-free culture | 0.98% | 1.60% | 1.27% | 0.82% |
| Cultured by the method of the present invention | 0.91% | 0.54% | 0.49% | 0.62% |

It can be seen that the expression of the positive index rate and the negative index rate of the MSC cell surface marker obtained by the culture method of the present invention is obviously better than the other two conventional culture methods.

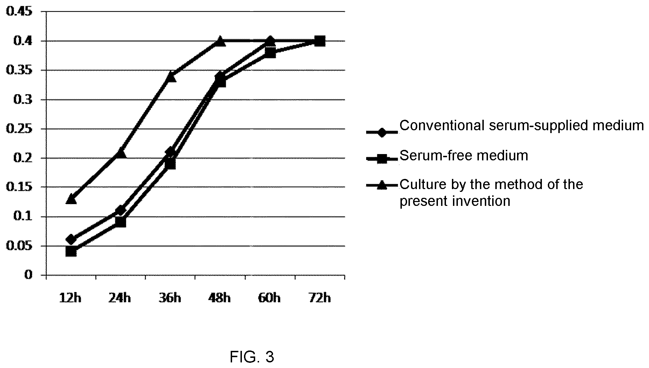

3.5 The comparison result of the cell proliferation rate of the MSCs obtained after being cultured for 14 d by the three culture methods respectively in the corresponding culture systems: as shown in FIG. 3, it can be seen that when the method of the present invention is used to culture MSCs, the cell proliferation rate is obviously better than the other two conventional culture methods.

3.6 Experimental results of the nude mice carcinogenicity test: no tumor formation could be seen in the two groups of mice which were respectively subcutaneously injected with 0.2 ml normal saline and 3×107/0.2ml MSCs to be cultured for 28 d during the 2 months of observation periods. The result indicates that after MSCs are cultured by the method of the present invention for 28 d, the MSCs are always safe and effective; and there is no tumor formation. The cultured and amplified MSCs do not form a tumor in vivo.

### Example 2

MSCs obtained by the method of the present invention in Example 1 were cryopreserved to compare the cryopreservation effect. Grouping was as follows:
Cryopreserving fluid 1: a 10 vol% DMSO and 90 vol% MesenPRO RS^{™} Medium;
Cryopreserving fluid 2: a 5 vol% DMSO and 90 vol% MesenPRO RS^{™} Medium;
Cryopreserving fluid 3: a 5 vol% DMSO and 10 vol% multiple electrolytes injection, and 75 vol% MesenPRO RS^{™} Medium;
Cryopreserving fluid 4: a 5 vol% DMSO and 10 vol% multiple electrolytes injection, and 10 vol% hydroxyethyl starch 200/0.5 sodium chloride injection, and 75 vol% MesenPRO RS^{™} Medium;
Cryopreserving fluid 5: a 5 vol% DMSO and 10 vol% multiple electrolytes injection, and 10 vol% hydroxyethyl starch 200/0.5 sodium chloride injection, 2 vol% albumin, and 73 vol% MesenPRO RS^{™} Medium;
Cryopreserving fluid 6 (the dedicated cryopreserving fluid of mesenchymal stem cells): a 5 vol% DMSO, 10% multiple electrolytes injection, 10 vol% hydroxyethyl starch 200/0.5 sodium chloride injection, 2 vol% albumin, 2 vol% hydroxylcamptothecine injection, and 71 vol% MesenPRO RS^{™} Medium;
MSC cells were cryopreserved by the following steps: the cryopreserving fluid was mixed with cells, and rapidly moved to a cryopreserved tube and put to a cryopreserved box, staying over the night at -80°C, and transferred into liquid nitrogen in the following day. 1 ml cryopreserving fluid was used every 5×107 MSC cells. MSC cells were cryopreserved for 90 d, and then resuscitated. The viability of the cells before and after cryopreservation, and the recovery rate of the cells after resuscitation were detected. Specifically, the calculation method for the viability of the cells before cryopreservation, after cryopreservation and resuscitation is as follows: [viable cell count/(viable cell count + dead cell count)] x 100%. The calculation method for the recovery rate of the cells after resuscitation is as follows: [viable cell count after resuscitation/(viable cell count when cryopreserved) x 100%.

The test result of the resuscitated MSC cells:

**Table 4 Comparison of cryopreservation and resuscitation effects among different cryopreserving fluids**

| | Cell viability (%) | Cell yield (%) |
|---|---|---|
| Cryopreserving fluid 1 | 91.12 | 89.38 |
| Cryopreserving fluid 2 | 92.86 | 90.17 |
| Cryopreservation solution 3 | 93.75 | 91.92 |
| Cryopreservation solution 4 | 95.63 | 93.49 |
| Cryopreserving fluid 5 | 96.17 | 94.99 |
| Cryopreserving fluid 6 | 99.35 | 97.11 |

As shown in Table 4, the cell viability after resuscitation is lower than that before cryopreservation. The viability and yield of the cells preserved by the cryopreserving fluid 2 are superior to those of cryopreserving fluid 1, indicating that 5% DMSO concentration is the most suitable concentration; the increase of the DMSO concentration will enhance the cytotoxicity of DMSO, but the MSC cell viability and cell viability may be not improved. The viability and yield of the cells preserved by the cryopreserving fluid 3 are superior to those of cryopreserving fluid 2, indicating that the multiple electrolytes injection has the effects of stabilizing and regulating crystal osmotic pressure, and may improve the viability and yield of the cryopreserved cells after resuscitation. The viability and yield of the cells preserved by the cryopreserving fluid 4 are superior to those of cryopreserving fluid 3, indicating that the hydroxyethyl starch 200/0.5 sodium chloride injection has the effects of stabilizing and regulating colloid osmotic pressure, and may improve the viability and yield of the cryopreserved cells after resuscitation. The viability and yield of the cells preserved by the cryopreserving fluid 5 are superior to those of cryopreserving fluid 4, indicating that the albumin has the effects of stabilizing and regulating colloid osmotic pressure, and may improve the viability and yield of the cryopreserved cells after resuscitation. The viability and yield of the cells preserved by the cryopreserving fluid 6 are superior to those of cryopreserving fluid 5, indicating that the hydroxylcamptothecine injection may improve the viability and yield of the cryopreserved cells after resuscitation. The viability and yield of the cells preserved by the cryopreserving fluid 6 are obviously superior to those of the rest 5 cryopreserving fluids; and the viability and yield of the MSCs after resuscitation are respectively up to 99% above and 97% above, indicating that the dedicated cryopreserving fluid of mesenchymal stem cells of the present invention is suitable for MSC cryopreservation.

### Example 3

The method for screening, activating, and amplifying mesenchymal stem cells in vitro in the examples of the present invention was used to obtain umbilical cord MSCs; then the MSCs were preserved by ABO/RH typing and HLA typing to establish a MSC information file for retrieval and to construct a mesenchymal stem cell bank.

### 1 Donor screening

The hospital consulted the donor's personal information, history of treatment, history of familial inheritance, and whether of history of infectious diseases, abnormal conditions of hematopoiesis or immune system and other information by inquiry and a way of filling a form. The hospital need to sign an informed consent with a donor to get the permission of the donor or its authorized officer, look up the physical examination data to obtain the physical examination information. Donor's physical examination information includes the following items: HIV antibody, hepatitis B surface antigen and antibody, hepatitis C antibody, cytomegalovirus antibody, treponema pallidum antibody, transaminase, and the like. Personal information acquisition table, informed consent, examination information and the like need to be numbered for seal preservation. The archival information base of umbilical cord donor for retrieval was established.

2 Specific preparation method includes the following steps: a 5 cm umbilical cord was set as an example and operated as follows (all the operation steps were completed in a clean bench):
2.1 Transportation of the umbilical cord: 5 cm umbilical cord was collected from a qualified donor passing through the screening and preserved in a vaccine box at a constant temperature of 2-8°C, and sent to a laboratory within 48 h.

2.2 Decomposition of the umbilical cord: the umbilical cord was taken and put to a 5 cm petri dish and washed, and cut into several segments, and the three middle blood vessels were removed. White connective tissue located between amniotic membrane and blood vessel is Whartonps jelly and torn down with long-handle toothed forceps, and put to a sterile dish. The Whartonps jelly was cut into about 1 mm3 tissue homogenate pieces in a centrifuge tube with sterile long-handle surgical scissors with a cut time of 15-20 min; 1 mg/ml II-type collagenase (preheated to 37°C) which had the same volume with the tissue homogenate piece was added, and placed to a constant temperature oscillation incubator for digestion for 60-90 min (taken out every 20 min and slightly shaken evenly and put them back) at 37°C and 200 rpm until tissue homogenate pieces of the umbilical cord were mylonitic. The digested tissue homogenate was added to 6-fold volume of normal saline according to a volume ratio of 1:6; residuals were removed with a 200-mesh filter screen; 400 g were centrifuged for 8 min, and supernatant was discarded to obtain the lower layer of cells.

2.3 Primary screening culture: based on cell counting, 50 ml dedicated MSC primary screening medium was added according to a density of 1.0×106/ml, namely, a Corning^{®} MSC Xeno-Free SFM added with 8ng/ml SCF, 4ng/ml BMP-4, 30IU/ml IL-10 and 4ng/ml LIF, 4ng/ml TGF- β, 8ng/ml rapamycin, 12ng/mlTrametinib, 20ng/ml paracetamol, 3ng/ml 5-HMF, 20ng/ml and chloroquine phosphate, and inoculated into 3 T175 culture flasks, then placed to a carbon dioxide incubator; the culture conditions were as follows: (37±0.5)°C, carbon dioxide volume fraction of (5±0.2)%, and medium change was performed once every 2-3 d.

2.4 Subculture activation and amplification: when the primary culture cells were up to 80% fusion on the 5-7 d around, the original cell culture solution was absorbed and discarded, and digested for subculture; after the subculture, the culture system was changed into a dedicated MSC activation and amplification medium, namely, the Corning^{®} MSC Xeno-Free SFM added with 8ng/ml SCF, 4ng/ml bFGF, 20ng/ml paeoniflorin, 30ng/ml metformin hydrochloride, 4ng/mlHydrocortisone, 4ng/ml CXCL10, 2ng/ml Forskolin, 3ng/ml 5-HMF and 20ng/ml chloroquine phosphate, and continuously cultured. The cells were observed every day and medium change was performed every 2-3 d according to the color of the medium.

3 Establishment of a cell bank: a dedicated cryopreserving fluid of mesenchymal stem cells was prepared by 5 vol% DMSO, 5% multiple electrolytes injection, 5 vol% hydroxyethyl starch 200/0.5 sodium chloride injection, 1 vol% albumin, 1 vol% hydroxylcamptothecine injection, and 83 vol% Corning^{®} MSC Xeno-Free SFM. The above activated and amplified umbilical cord MSCs were collected. MSC cell suspension was subjected to the following examination: mycoplasma, endotoxin, microorganism and virus (five items). Qualified MSCs were detected, and 1 ml of the above prepared dedicated MSC cryopreserving fluid was used for every 5×107 qualified MSC cells for cryopreserving operation, and preserved by ABO/RH typing and HLA typing, thus establishing an information file of umbilical cord mesenchymal stem cells for retrieval to construct a mesenchymal stem cell bank.

### Example 4

The method for screening, activating, and amplifying mesenchymal stem cells in vitro in the examples of the present invention was used to obtain placenta MSCs; then the MSCs were preserved by ABO/RH typing and HLA typing to establish a placenta MSC information file for retrieval and to construct a placenta mesenchymal stem cell bank.

### 1 Donor screening

The hospital consulted the donor's personal information, history of treatment, history of familial inheritance, and whether of history of infectious diseases, abnormal conditions of hematopoiesis or immune system and other information by inquiry and a way of filling a form. The hospital need to sign an informed consent with a donor to get the permission of the donor or its authorized officer, look up the physical examination data to obtain the physical examination information. Donor's physical examination information includes the following items: HIV antibody, hepatitis B surface antigen and antibody, hepatitis C antibody, cytomegalovirus antibody, Treponema pallidum antibody, transaminase, and the like. Personal information acquisition table, informed consent, examination information and the like need to be numbered for seal preservation. The archival information base of placenta donor for retrieval was established.

### 2 Preparation of placenta MSCs

2.1 A copy of intact placenta from a qualified donor was collected, and added with a proper amount of placenta preserving fluid (obtained by dissolving 100 mg penicillin, 100 mg streptomycin, 2.5 mg amphotericin, 0.5 g heparin sodium injection and 2.5 g human serum albumin into a 500ml DMEM medium) until the placenta was immersed, then preserved into a vaccine box at a constant temperature of 2-8°C, and sent to a laboratory within 24 h.

2.2 The placenta preserving fluid was absorbed and discarded; a proper volume of normal saline containing double antibodies was added, and repeatedly oscillated for several times until the blood stain on the surface of the placenta was cleaned. Amniotic membrane and decidua on the surface of placenta were removed; then a proper volume of normal saline containing double antibodies was added, and the placenta tissue was cut into pieces, and blood in the tissue block was cleaned.

2.3 Digestion of placenta: the placenta tissue block which was cut into pieces was collected; DMEM (collagenase IV with a final concentration of 0.1%) which had the same volume with the placenta tissue block and contained 0.2% IV collagenase was added, and then digested for 20-30 min in a shaking bath at 37°C, then DMEM which had 1/4 volume of the placenta tissue block and contained 0.25% trypsin was added and digested for 10-20 min in a shaking bath at 37°C. The placenta tissue block digested for twice was filtered with a 200-mesh analysis sieve to collect filtrate; mixed well and centrifuged for 10 min at 1800 rpm and room temperature, then supernatant was discarded; a proper amount of normal saline was used to resuspend cell precipitates; HES was added according to a volume ratio of resuspending solution: HES of 4:1, and centrifuged for 10 min at 300 rpm, and supernatant liquid was collected and centrifuged to obtain cells.

2.4 Primary screening culture: based on cell counting, 50 ml dedicated MSC primary screening medium was added according to a density of 1.0×106/ml, namely, a MesenPRO RS^{™} Medium added with 2ng/ml SCF, 2ng/ml BMP-4, 10IU/ml IL-10 and 1ng/ml LIF, 1ng/ml TGF-β , 2ng/ml rapamycin, 2ng/mlTrametinib, 10ng/ml paracetamol, 1ng/ml 5-HMF and 10ng/ml chloroquine phosphate, and inoculated to 3 T175 culture flasks, then placed to a carbon dioxide incubator; the culture conditions were as follows: (37±0.5)°C, carbon dioxide volume fraction of (5±0.2)%, and medium change was performed once every 2-3 d.

2.5 Subculture activation and amplification: when the primary culture cells were up to 80% fusion on the 5-7 d around, the original cell culture solution was absorbed and discarded, and digested for subculture; after the subculture, the culture system was changed into a dedicated MSC activation and amplification medium, namely, the MesenPRO RS^{™} Medium added with 2ng/ml SCF, 1-4ng/ml bFGF, 10ng/ml paeoniflorin, 20ng/ml metformin hydrochloride, 1ng/mlHydrocortisone, 2ng/ml CXCL10, 1ng/ml Forskolin, 1ng/ml 5-HMF and 10ng/ml chloroquine phosphate, and continuously cultured. The cells were observed every day and medium change was performed every 2-3 d according to the color of the medium.

3 Establishment of a cell bank: a dedicated cryopreserving fluid of mesenchymal stem cells was prepared by a 5 vol% DMSO, 10% multiple electrolytes injection, 10 vol% hydroxyethyl starch 200/0.5 sodium chloride injection, 2 vol% albumin, 2 vol% hydroxylcamptothecine injection, and 71 vol% MesenPRO RS^{™} Medium. The above activated and amplified placenta MSCs were collected. MSC cell suspension was subjected to the following examination: mycoplasma, endotoxin, microorganism and virus (five items). 1 ml of the above prepared dedicated MSC cryopreserving fluid was used for each 5×107 qualified MSC cells for cryopreserving operation, and the cells were preserved by ABO/RH typing and HLA typing, thus establishing a placenta MSC information file for retrieval to construct a mesenchymal stem cell bank.

## Claims

1. A method for screening, activating, amplifying and cryopreserving mesenchymal stem cells in vitro and establishing a cell bank of mesenchymal stem cells, comprising: using a dedicated primary screening medium of mesenchymal stem cells for first-stage screening culture to obtain purified mesenchymal stem cells; wherein the dedicated primary screening medium of mesenchymal stem cells is a serum-free complete medium of mesenchymal stem cells added with 2-8 ng/ml SCF, 2-4 ng/ml BMP-4, 10-30 IU/ml IL-10 and 1-4 ng/ml LIF, 1-4 ng/ml TGF-β, 2-8 ng/ml rapamycin, 2-12 ng/ml trametinib, 10-20 ng/ml paracetamol, 1-3 ng/ml 5-HMF and 10-20 ng/ml chloroquine phosphate; using a dedicated activation and amplification medium of mesenchymal stem cells to perform second-stage activation and amplification culture on the purified mesenchymal stem cells wherein cells are subcultured once every 2-3 d for more than once to obtain a large number of mesenchymal stem cells with activation functions; using a dedicated cryopreserving fluid of mesenchymal stem cells to cryopreserve the stem cells and performing preservation according to ABO/RH typing and HLA typing; and establishing an information file for retrieval to construct a mesenchymal stem cell bank.

2. The method according to claim 1, wherein the dedicated activation and amplification medium of mesenchymal stem cells is a serum-free complete medium of mesenchymal stem cells added with 2-8 ng/ml SCF, 1-4 ng/ml bFGF, 10-20 ng/ml paeoniflorin, 20-30 ng/ml metformin hydrochloride, 1-4 ng/ml hydrocortisone, 2-4 ng/ml CXCL10, 1-2 ng/ml Forskolin, 1-3 ng/ml 5-HMF, and 10-20 ng/ml chloroquine phosphate.

3. The method according to claim 1, wherein the dedicated cryopreserving fluid of mesenchymal stem cells is a 66-83 vol% serum-free complete medium of mesenchymal stem cells containing 5-10 vol% DMSO, 5-10 vol% multiple electrolytes injection, 5-10 vol% hydroxyethyl starch 200/0.5 sodium chloride injection, 1-2 vol% albumin, and 1-2 vol% hydroxylcamptothecine injection .

4. The method according to any one of claims 1, 2 and 3, wherein in the dedicated primary screening medium of mesenchymal stem cells, the dedicated activation and amplification medium of mesenchymal stem cells and the dedicated cryopreserving fluid of mesenchymal stem cells, the serum-free complete medium is a commercially available serum-free media.

## Patentansprüche

1. - Verfahren zum Screening, Aktivieren, Proliferieren und Kryokonservieren von mesenchymalen Stammzellen *in vitro* und zum Erstellen einer Zellbank von mesenchymalen Stammzellen, umfassend: Verwenden eines dedizierten primären Screening-Mediums für mesenchymale Stammzellen für eine Screening-Kultur der ersten Stufe, um aufgereinigte mesenchymale Stammzellen zu erlangen; wobei das dedizierte primäre Screening-Medium für mesenchymale Stammzellen ein serumfreies vollständiges Medium für mesenchymale Stammzellen ist, zu dem 2-8 ng/ml SCF, 2-4 ng/ml BMP-4, 10-30 IU/ml IL-10 und 1-4 ng/ml LIF, 1-4 ng/ml TGF-β, 2-8 ng/ml Rapamycin, 2-12 ng/ml Trametinib, 10-20 ng/ml Paracetamol, 1-3 ng/ml 5-HMF und 10-20 ng/ml Chloroquinphosphat hinzugefügt werden; Verwenden eines dedizierten Aktivierungs- und Proliferationsmediums für mesenchymale Stammzellen zum Durchführen einer Aktivierungs- und Proliferationskultur der zweiten Stufe an den aufgereinigten mesenchymalen Stammzellen, wobei die Zellen einmal alle 2-3 Tage mehr als einmal subkultiviert werden, um eine große Anzahl mesenchymaler Stammzellen mit Aktivierungsfunktionen zu erlangen; Verwenden einer speziellen Kryokonservierungsflüssigkeit für mesenchymale Stammzellen zum Kryokonservieren der Stammzellen und Durchführen der Konservierung gemäß ABO/RH-Typisierung und HLA-Typisierung; und Erstellen einer Informationsdatei zum Abruf, um eine Bank mesenchymaler Stammzellen aufzubauen.

2. - Verfahren nach Anspruch 1, wobei das dedizierte Aktivierungs- und Proliferationsmedium für mesenchymale Stammzellen ein serumfreies vollständiges Medium für mesenchymale Stammzellen ist, zu dem 2-8 ng/ml SCF, 1-4 ng/ml bFGF, 10-20 ng/ml Paeoniflorin, 20-30 ng/ml Metforminhydrochlorid, 1-4 ng/ml Hydrocortison, 2-4 ng/ml CXCL10, 1-2 ng/ml Forskolin, 1-3 ng/ml 5-HMF und 10-20 ng/ml Chloroquinphosphat hinzugefügt werden.

3. - Verfahren nach Anspruch 1, wobei die dedizierte Kryokonservierungsflüssigkeit für mesenchymale Stammzellen ein 66-83 Volumen-% serumfreies vollständiges Medium für mesenchymale Stammzellen ist, das 5-10 Volumen-% DMSO, 5-10 Volumen-% Multiple-Elektrolyte-Injektion, 5-10 Volumen-% Hydroxyethylstärke-200/0, 5-Natriumchlorid-Injektion, 1-2 Volumen-% Albumin und 1-2 Volumen-% Hydroxylcamptothecin-Injektion enthält.

4. - Verfahren nach einem der Ansprüche 1, 2 und 3, wobei in dem dedizierten primären Screening-Medium für mesenchymale Stammzellen, dem dedizierten Aktivierungs- und Proliferationsmedium für mesenchymale Stammzellen und der dedizierten Kryokonservierungsflüssigkeit für mesenchymale Stammzellen das serumfreie vollständige Medium ein im Handel erhältliches serumfreies Medium ist.

## Revendications

1. - Procédé de criblage, d'activation, de prolifération et de cryoconservation de cellules souches mésenchymateuses in vitro et d'établissement d'une banque de cellules souches mésenchymateuses, comprenant : l'utilisation d'un milieu dédié au criblage primaire des cellules souches mésenchymateuses pour une culture de criblage de première étape pour obtenir des cellules souches mésenchymateuses purifiées ; dans lequel le milieu dédié au criblage primaire des cellules souches mésenchymateuses est un milieu complet sans sérum de cellules souches mésenchymateuses auquel sont ajoutés 2 à 8 ng/ml de SCF, 2 à 4 ng/ml de BMP-4, 10 à 30 UI/ml d'IL-10 et 1 à 4 ng/ml de LIF, 1 à 4 ng/ml de TGF-β, 2 à 8 ng/ml de rapamycine, 2 à 12 ng/ml de tramétinib, 10 à 20 ng/ml de paracétamol, 1 à 3 ng/ml de 5-HMF et 10 à 20 ng/ml de phosphate de chloroquine ; l'utilisation d'un milieu dédié à l'activation et à la prolifération des cellules souches mésenchymateuses pour réaliser une culture d'activation et de prolifération de deuxième étape sur les cellules souches mésenchymateuses purifiées dans lequel les cellules sont repiquées une fois tous les 2 à 3 jours à plusieurs reprises pour obtenir un grand nombre de cellules souches mésenchymateuses avec des fonctions d'activation ; l'utilisation d'un fluide dédié à la cryoconservation des cellules souches mésenchymateuses pour cryoconserver les cellules souches et réaliser la conservation selon le typage ABO/RH et le typage HLA ; et l'établissement d'un fichier d'informations pour la récupération afin de construire une banque de cellules souches mésenchymateuses.

2. - Procédé selon la revendication 1, dans lequel le milieu dédié à l'activation et à la prolifération des cellules souches mésenchymateuses est un milieu complet sans sérum de cellules souches mésenchymateuses auquel sont ajoutés 2 à 8 ng/ml de SCF, 1 à 4 ng/ml de bFGF, 10 à 20 ng/ml de paéoniflorine, 20 à 30 ng/ml de chlorhydrate de metformine, 1 à 4 ng/ml d'hydrocortisone, 2 à 4 ng/ml de CXCL10, 1 à 2 ng/ml de forskoline, 1 à 3 ng/ml de 5-HMF et 10 à 20 ng/ml de phosphate de chloroquine.

3. - Procédé selon la revendication 1, dans lequel le fluide dédié à la cryoconservation des cellules souches mésenchymateuses est un milieu complet sans sérum à 66 à 83 % en volume de cellules souches mésenchymateuses contenant 5 à 10 % en volume de DMSO, 5 à 10 % en volume d'injection d'électrolytes multiples, 5 à 10 % en volume d'injection d'hydroxyéthylamidon 200/0,5 chlorure de sodium, 1 à 2 % en volume d'albumine et 1 à 2 % en volume d'injection d'hydroxycamptothécine.

4. - Procédé selon l'une quelconque des revendications 1, 2 et 3, dans lequel dans le milieu dédié au criblage primaire des cellules souches mésenchymateuses, le milieu dédié à l'activation et à la prolifération des cellules souches mésenchymateuses et le fluide dédié à la cryoconservation des cellules souches mésenchymateuses, le milieu complet sans sérum est un milieu sans sérum disponible dans le commerce.
